# FASCICULE DE BREVET EUROPEEN

(11) **EP 0 775 213 B1**
(45) Date de publication et mention de la délivrance du brevet: **20.04.2005**
(21) Numéro de dépôt: 95926429.2
(22) Date de dépôt: 26.07.1995
(51) Int. Cl.: C12N 15/86, A61K 48/00, C12N 9/08, A61K 38/43

(54) **ADENOVIRUS COMPRENANT UN GENE CODANT POUR LA GLUTATHION PEROXYDASE**
ADENOVIRUS MIT GLUTATHION PEROXYDATE GENE
ADENOVIRUS COMPRISING A GENE CODING FOR GLUTATHION PEROXIDASE

(30) Priorité: 12.08.1994 FR 9409982
(43) Date de publication de la demande: 28.05.1997
(73) Titulaire: Aventis Pharma S.A., 92160 Antony (FR)
(72) Inventeur: BARKATS, Martine, F-75005 Paris (FR); MALLET, Jacques, F-75013 Paris (FR); REVAH, Frédéric, F-92160 Antony (FR)
(74) Mandataire: Gallois, Valérie
(86) Numéro de dépôt international: PCT/FR1995/001002
(87) Numéro de publication internationale: WO 1996/005320

(56) Documents cités:
- WO-A-88/07541
- WO-A-90/06757
- WO-A-93/20195
- NUCLEIC ACIDS RESEARCH, vol. 21, ARLINGTON, VIRGINIA US, page 1607 ERZURUM SC ET AL 'PROTECTION OF HUMAN ENDOTHELIAL-CELLS FROM OXIDANT INJURY BY ADENOVIRUS-MEDIATED TRANSFER OF THE HUMAN CATALASE CDNA'
- BRAIN PATHOLOGY, vol. 4, page 3 AGUZZI A ET AL 'TRANSGENIC AND KNOCK-OUT MICE - MODELS OF NEUROLOGICAL DISEASE'
- CHEMICAL ABSTRACTS, vol. 105, no. 19, 10 Novembre 1986 Columbus, Ohio, US; abstract no. 166105, BELL, G. I. ET AL 'cDNA sequence coding for human kidney catalase' & NUCLEIC ACIDS RES. (1986), 14(13), 5561-2 CODEN: NARHAD;ISSN: 0305-1048,
- MEDECINE/SCIENCE, vol. 9, page 208 O.DANOS ET AL. 'REEIMPLANTATION de cellules génétiquement modifiées dans des néo-organes vascularisés'
- MICHIELS ET AL: 'Use of the inhibition of enzymatic antioxidant systems in order to evaluate their physiological importance' EUR. J. BIOCHEISTRY 1988, pages 435 - 441

## Description

La présente invention concerne des adénovirus recombinants comprenant une séquence d'ADN codant pour la glutathion peroxydase et ses utilisations en thérapie génique.

La glutathion peroxydase est une des enzymes qui intervient activement dans la régulation des concentrations en radicaux libres dérivés de l'oxygène, formés lors de divers processus physiologiques ou pathologiques.

Normalement, la formation de ces radicaux, hautement réactifs, comme l'anion superoxyde, le peroxyde d'hydrogène et le radical hydroxyle est contrôlée comme suit: l'anion superoxyde est rapidement converti en peroxyde d'hydrogène, gràce à la superoxyde dismutase, puis ce peroxyde d'hydrogène est converti en oxygène et eau, par la catalase ou en particulier la glutathion peroxydase.

Habituellement, ces enzymes sont présentes dans pratiquement tous les tissus.

Toutefois, sous certaines conditions, ces mécanismes de régulation ne sont pas totalement efficaces. En particulier, il peut exister un déséquilibre entre leurs concentrations respectives, par exemple une concentration excessive en superoxyde dismutase comparativement à la quantité disponible de glutathion peroxydase, ce qui conduit à une production pathologique de peroxyde d'hydrogène et de radicaux libres (radicaux hydroxyle en particulier).

Ces radicaux libres peuvent directement induire une peroxydation des lipides menbranaires, inactiver les enzymes en peroxydant leurs groupements sulfhydryle, dépolymériser des polysaccharides et/ou endommager des acides nucléiques, entrainant dans tous les cas des pathologies graves. Ils peuvent être ainsi à l'origine d'inflammations, d'emphysèmes, de néoplasmes et/ou de rétinopathies. Ils semblent également être impliqués dans l'athérosclérose, l'ischémie cérébrale, les traumatismes crâniens, le syndrome de détresse respiratoire, les maladies cardiovasculaires, le diabète, la cirrhose du foie et la formation de cataractes ainsi que dans le processus de vieillissement. Les radicaux libres seraient également liés au processus d'apoptose et pourraient intervenir dans la mort cellulaire accompagnant le syndrome d'immunodéficience acquise (SIDA), [The J. of Biol. Chem., 269, 2(14), 798-801, (1994).] Plus récemment, il a été mis en évidence que des réactions entre ces radicaux ou avec des neurotransmetteurs conduisaient à la formation de neurotoxines endogènes. Les radicaux libres sont donc également impliqués dans des pathologies neurologiques comme la maladie d'Alzheimer, la maladie de Parkinson, la sclérose latérale amyotrophique (ALS) et/ou la trisomie 21.

En conséquence, il serait particulièrement précieux de disposer aujourd'hui de médicaments qui puissent accroître ou réguler la concentration en glutathion peroxydase au niveau de l'organisme et qui soient donc efficaces pour traiter l'ensemble des pathologies précitées.

Erzurum et al., 1993, Nucleic Acids Research, vol. 21, No. 7 décrit l'utilisation d'un adénovirus défectif porteur de l'ADNc de la catalase humaine pour le transfert de la catalase dans les cellules endothéliales.

La présente invention réside précisément dans la mise au point de vecteurs particulièrement efficaces pour délivrer in vivo et de manière localisée, des quantités thérapeutiquement actives du gène spécifique codant pour la glutathion peroxydase.

Dans la demande correspondante n° PCT/EP93/02519, il a été montré que les adénovirus pouvaient être utilisés comme vecteur pour le transfert d'un gène étranger in vivo dans le système nerveux et l'expression de la protéine correspondante.

La présente invention concerne plus particulièrement des constructions nouvelles, particulièrement adaptées et efficaces pour contrôler l'expression de la glutathion peroxydase.

Plus précisément, elle se rapporte à un adénovirus recombinant comprenant une séquence d'ADN propre à contrôler l'expression d'une glutathion peroxydase, son utilisation pour des traitements thérapeutiques et/ou la prévention de diverses pathologies.

La demanderesse a ainsi mis en évidence qu'il est possible de construire des adénovirus recombinants contenant une séquence codant pour une glutathion peroxydase, d'administrer ces adénovirus recombinants in vivo, et que cette administration permet une expression stable et localisée de quantités thérapeutiquement actives de la glutathion peroxydase in vivo.

Un premier objet de l'invention réside donc dans un adénovirus recombinant défectif comprenant au moins une séquence d'ADN codant pour tout ou une partie active d'une glutathion peroxydase.

Au sens de la présente invention on entend désigner par glutathion peroxydase toute enzyme possédant l'activité de la glutathion peroxydase. A titre illustratif de ces enzymes on peut en particulier citer chez l'homme les glutathions peroxydases, GPX1, GPX2, GPX3 et GPX4. Les GPX1et GPX4 sont exprimées dans la plupart des tissus avec une nette prépondérance dans les érythrocytes, le foie et le rein, pour GPX1(Chambers et al; EMBO J 5: 1221-1227 (1986)) et les testicules pour GPX4 [Roveri et al; J. Biol. Chem. 267:6142-6146 (1992)]. GPX3 est produite dans le rein, le poumon, le coeur, le sein, le placenta ainsi que dans le foie (Chu et al. Blood 79: 3233_3238 (1992)) quant à la GPX2, elle a principalement été mise en évidence dans les tissus gastrointestinaux et dans le foie [Chu et al. J. Biol. Chem. 268: 2571-257 (1993)].

La glutathion peroxydase produite dans le cadre de la présente invention peut être une glutathion peroxydase humaine ou animale. Il peut en particulier s'agir de la glutathion peroxydase bovine.

La séquence d'ADN codant pour la glutathioa peroxydase, utilisée dans le cadre de la présente invention peut être un ADNc, un ADN gértomique (ADNg), ou une construction hybride consistant par exemple en un ADNc dans lequel seraient insérés un ou plusieurs mirons. La séquence nucléique de l'ADNc codant pour la glutathion peroxydase humaine a été décrite par [Mullenbach et al., Oxy-Radicals in Molecular Biology and Pathology, 313-326, (1988)]. Il peut également s'agir de séquences synthétiques ou semisynthétiques.

De manière particulièrement avantageuse, on utilise un ADNc ou un ADNg.

Selon un mode préféré de l'invention, il s'agit une séquence d'ADN génomique (ADNg) codant pour une glutathion peroxydase. Son utilisation peut permettre une meilleure expression dans les cellules humaines.

Bien entendu, préalablement à son incorporation dans un vecteur adénovirus selon l'invention, la séquence d'ADN peut être avantageusement modifiée, par exemple par mutagénèse dirigée, en particulier pour l'insertion de sites de restriction appropriés. Les séquences décrites dans l'art antérieur ne sont en effet pas construites pour une utilisation selon l'invention, et des adaptations préalables peuvent s'avérer nécessaires, pour obtenir des expressions importantes.

La séquence d'ADN, codant pour tout ou partie d'une glutathion peroxydase , peut également être une séquence antisens, dont l'expression dans la cellule cible permet de Contrôler l'expression de cette enzyme. Préférentiellement, la séquence d'ADN hétérologue comporte un gène codant pour un ARN antisens capable de contrôler la traduction de l'ARNm correspondant. La séquence antisens peut être tout ou seulement une partie de la séquence d'ADN, codant pour une glutathion peroxydase, insérée dans l'orientation inverse dans le vecteur selon l'invention.

Selon un mode de réalisation de l'invention, la séquence d'ADN, codant pour une glutathion peroxydase, peut également intégrer un signal de sécrétion permettant de diriger la glutathion peroxydase synthétisée dans les voies de sécrétion des cellules infectées. De cette manière, la glutathion peroxydase synthétisée est avantageusement libérée dans les compartiments extracellulaires.

Avantageusement, la séquence codant pour la glutathion peroxydase est placée sous le contrôle de signaux permettant son expression dans les cellules cibles. Préférentiellement, il s'agit de signaux d'expression hétérologues, c'est-à-dire de signaux différents de ceux naturellement responsables de l'expression de la glutathion peroxydase. Il peut s'agir en particulier de séquences responsables de l'expression d'autres protéines, ou de séquences synthétiques. Notamment, il peut s'agir de séquences promotrices de gènes eucaryotes ou viraux. Par exemple, il peut s'agir de séquences promotrices issues du génome de la cellule que l'on désire infecter. De même, il peut s'agir de séquences promotrices issues du génome d'un virus, y compris l'adénovirus utilisé. A cet égard, on peut citer par exemple les promoteurs E1A, MLP, CMV, LTR-RSV, etc. En outre, ces séquences d'expression peuvent être modifiées par addition de séquences d'activation, de régulation, ou permettant une expression tissu-spécifique. Il peut en effet être particulièrement intéressant d'utiliser des signaux d'expression actifs spécifiquement ou majoritairement dans les cellules cibles, de manière à ce que la séquence d'ADN ne soit exprimée et ne produise son effet que lorsque le virus a effectivement infecté une cellule cible.

Dans un premier mode de réalisation particulier, l'invention concerne un adénovirus recombinant défectif comprenant une séquence d'ADNc ou ADN8 codant pour une glutathion peroxydase bovine sous le contrôle du promoteur LTR-RSV.

Dans un autre mode de réalisation particulier, l'invention concerne un adénovirus recombinant défectif comprenant une séquence d'ADNg codant pour la glutathion peroxydase humaine sous le contrôle du promoteur LTR-RSV.

Un mode particulièrement préféré de mise en oeuvre de la présente invention réside dans un adénovirus recombinant défectif comprenant les séquences ITR, une séquence permettant l'encapsidation, une séquence d'ADN codant pour la glutathion peroxydase humaine sous le contrôle d'un promoteur permettant une expression majoritaire dans les tissus cibles et dans lequel le gène E1 et au moins un des gènes E2, E4, L1-L5 est non fonctionnel.

Les adénovirus défectifs selon l'invention sont des adénovirus incapables de se répliquer de façon autonome dans la cellule cible. Généralement, le génome des adénovirus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par la séquence d'ADN codant pour la glutathion peroxydase.

Préférentiellement, le virus défectif de l'invention conserve les séquences de son génome qui sont nécessaires à l'encapsidation des particules virales. Encore plus préférentiellement, comme indiqué ci-avant, le génome du virus recombinant défectif selon l'invention comprend les séquences ITR, une séquence permettant l'encapsidation, le gène E1 non fonctionnel et au moins un des gènes E2, E4, L1-L5 non fonctionnel.

n existe différents sérotypes d'adénovirus, dont la structure et les propriétés varient quelque peu. Parmi ces sérotypes, on préfère utiliser dans le cadre de la présente invention les adénovirus humains de type 2 ou 5 (Ad 2 ou Ad 5) ou les adénovirus d'origine animale (voir demande FR 93 05954). Parmi les adénovirus d'origine animale utilisables dans le cadre de la présente invention on peut citer les adénovirus d'origine canine, bovine, murine, [exemple : Mavl, Beard et al., Virology 75 (1990) 81], ovine, porcine, aviaire ou encore simienne (exemple : SAV). De préférence, l'adénovirus d'origine animale est un adénovirus canin, plus préférentiellement un adénovirus CAV2 [souche manhattan ou A26/61 (ATCC VR-800) par exemple]. De préférence, on utilise dans le cadre de l'invention des adénovirus d'origine humaine ou canine ou mixte.

Les adénovirus recombinants défectifs selon l'invention peuvent être préparés par toute technique connue de l'homme du métier (Levrero et al., Gene 101 (1991) 195, EP 185 573; Graham, EMBO J. 3 (1984) 2917). En particulier, ils peuvent être préparés par recombinaison homologue entre un adénovirus et un plasmide portant entre autre la séquence d'ADN codant pour la glutathion peroxydase. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %). Des stratégies de construction de vecteurs dérivés des adénovirus ont également été décrites dans les demandes n° FR 93 05954 et FR 93 08596 qui sont incorporées à la présente demande par référence.

Ensuite, les adénovirus qui se sont multipliés sont récupérés et purifiés selon les techniques classiques de biologie moléculaire, comme illustré dans les exemples.

Les propriétés particulièrement avantageuses des vecteurs de l'invention découlent notamment de la construction utilisée (adénovirus défectif, délété de certaines régions virales), du promoteur utilisé pour l'expression de la séquence codant pour la glutathion peroxydase (promoteur viral ou tissu-spécifique de préférence), et des méthodes d'aministration dudit vecteur, permettant l'expression efficace et dans les tissus appropriés de ladite enzyme.

La présente invention concerne également toute utilisation d'un adénovirus tel que décrit ci-dessus pour la préparation d'une composition pharmaceutique destinée au traitement et/ou à la prévention des pathologies précédemment citées. Plus particulièrement, elle concerne toute utilisation de ces adénovirus pour la préparation d'une composition pharmaceutique destinée au traitement et/ou à la prévention des maladies neurodégénératives comme par exemple la maladie de Parkinson, la maladie d'Alzheimer, et la sclérose latérale amyotrophique (ALS).

La présente invention concerne également une composition pharmaceutique comprenant un ou plusieurs adénovirus recombinants défectifs tels que décrits précédemment. Ces compositions pharmaceutiques peuvent être formulées en vue d'administrations par voie topique, orale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe chez le patient. Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables.

A cet égard, l'invention concerne également une méthode de traitement des maladies neurodégénératives comprenant l'administration à un patient d'un adénovirus recombinant tel que défini ci-avant. Plus particulièrement, l'invention concerne une méthode de traitement des maladies neurodégénératives comprenant l'administration stéréotaxique d'un adénovirus recombinant tel que défini ci-avant.

Les doses d'adénovirus recombinant défectif utilisées pour l'injection peuvent être adaptées en fonction de différents paramètres, et notamment en fonction du mode d'administration utilisé, de la pathologie concernée ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, puis mesure, généralement après 48 heures, du nombre de plages de cellules infectées. Les techniques de détermination du titre pfu d'une solution virale sont bien documentées dans la littérature.

Un autre objet de l'invention concerne toute cellule isolée de mammifère infectée par un ou plusieurs adénovirus recombinants défectifs tels que décrits ci-dessus. Plus particulièrement, l'invention concerne toute population de cellules humaines isolées infectée par ces adénovirus. Il peut s'agir en particulier de fibroblastes, myoblastes, hépatocytes, kératinocytes, cellules endothéliales, cellules Gliales, etc.

Les cellules selon l'invention peuvent être issues de cultures primaires. Celles-ci peuvent être prélevées par toute technique connue de l'homme du métier, puis mises en culture dans des conditions permettant leur prolifération. S'agissant plus particulièrement de fibroblastes, ceux-ci peuvent être aisément obtenus à partir de biopsies, par exemple selon la technique décrite par Ham [Methods Cell.Biol. 21a (1980) 255]. Ces cellules peuvent être utilisées directement pour l'infection par les adénovirus, ou conservées, par exemple par congélation, pour l'établissement de banques autologues, en vue d'une utilisation ultérieure. Les cellules selon l'invention peuvent également être des cultures secondaires, obtenues par exemple à partir de banques préétablies.

Les cellules en culture sont ensuite infectées par des adénovirus recombinants, pour leur conférer la capacité de produire de la glutathion peroxydase. L'infection est réalisée in vitro selon des techniques connues de l'homme du métier. En particulier, selon le type de cellules utilisé et le nombre de copies de virus par cellule désiré, l'homme du métier peut adapter la multiplicité d'infection. Il est bien entendu que ces étapes doivent être effectuées dans des conditions de stérilité appropriées lorsque les cellules sont destinées à une administration in vivo. Les doses d'adénovirus recombinant utilisées pour l'infection des cellules peuvent être adaptées par l'homme du métier selon le but recherché. Les conditions décrites ci-avant pour l'administration in vivo peuvent être appliquées à l'infection in vitro.

Un autre objet de l'invention concerne un implant comprenant des cellules mammifère infectées par un ou plusieurs adénovirus recombinants défectifs telles que décrites ci-dessus, et une matrice exuacellulaire. Préférentiellement, les implants selon l'invention comprennent 10⁵ à 10¹⁰ cellules. Plus préférentiellement, ils en comprennent 10⁶ à 10⁸.

Plus particulièrement, dans les implants de l'invention, la matrice extracellulaire comprend un composé gélifiant et éventuellement un support permettant l'ancrage des cellules.

Pour la préparation des implants selon l'invention. différents types de gélifiants peuvent être employés. Les gélifiants sont utilisés pour l'inclusion des cellules dans une matrice ayant la constitution d'un gel, et pour favoriser l'ancrage des cellules sur le support, le cas échéant. Différents agents d'adhésion cellulaire peuvent donc être utilisés comme gélifiants, tels que notamment le collagène, la gélatine, les glycosaminoglycans, la fibronectine, les lectines, l'agarose etc.

Comme indiqué ci-avant, les compositions selon l'invention comprennent avantageusement un support permettant l'ancrage des cellules. Le terme ancrage désigne toute forme d'interaction biologique et/ou chimique et/ou physique entraînant l'adhésion et/ou la fixation des cellules sur le support. Par ailleurs, les cellules peuvent soit recouvrir le support utilisé, soit pénétrer à l'intérieur de ce support, soit les deux. On préfère utiliser dans le cadre de l'invention un support solide, non toxique et/ou bio-compatible. En particulier, on peut utiliser des fibres de polytétrafluoroéthylène (PTFE) ou un support d'origine biologique.

Les implants selon l'invention peuvent être implantés en différents sites de l'organisme. En particulier, l'implantation peut être effectuée au niveau de la cavité péritonéale, dans le tissu sous-cutané (région sus-pubienne, fosses iliaques ou inguinales, etc), dans un organe, un muscle, une tumeur, le système nerveux central, ou encore sous une muqueuse. Les implants selon l'invention sont particulièrement avantageux en ce sens qu'ils permettent de contrôler la libération du produit thérapeutique dans l'organisme: Celle-ci est tout d'abord déterminée par la multiplicité d'infection et par le nombre de cellules implantées. Ensuite, la libération peut être contrôlée soit par le retrait de l'implant, ce qui arrête définitivement le traitement, soit par l'utilisation de systèmes d'expression régulable, permettant d'induire ou de réprimer l'expression des gènes thérapeutiques.

La présente invention fournit ainsi des vecteurs viraux utilisables directement en thérapie génique, particulièrement adaptés et efficaces pour diriger l'expression de la glutathion peroxydase in vivo. La présente invention offre ainsi une nouvelle approche particulièrement avantageuse pour le traitement et/ou la prévention de nombreuses pathologies comme celles citées précédemment.

Les vecteurs adénoviraux selon l'invention présentent en outre des avantages importants, liés notamment à leur très haute efficacité d'infection des cellules cibles, permettant de réaliser des infections à partir de faibles volumes de suspension virale. De plus, l'infection par les adénovirus de l'invention est très localisée au site d'injection, ce qui évite les risques de diffusion aux structures cérébrales voisines. Ce traitement peut concerner aussi bien l'homme que tout animal tel que les ovins, les bovins, les murins, les animaux domestiques (chiens, chats, etc), les chevaux, les poissons, etc.

On peut en outre, parfaitement envisager de procéder à une administration conjointe d'un adénovirus selon l'invention avec au moins un second adénovirus comportant un gène codant pour une des formes de la superoxyde dismutase ou la catalase.

Les exemples et la figure unique sont présentés ci-après à titre illustratif et non limitatif du domaine de l'invention.

### FIGURE

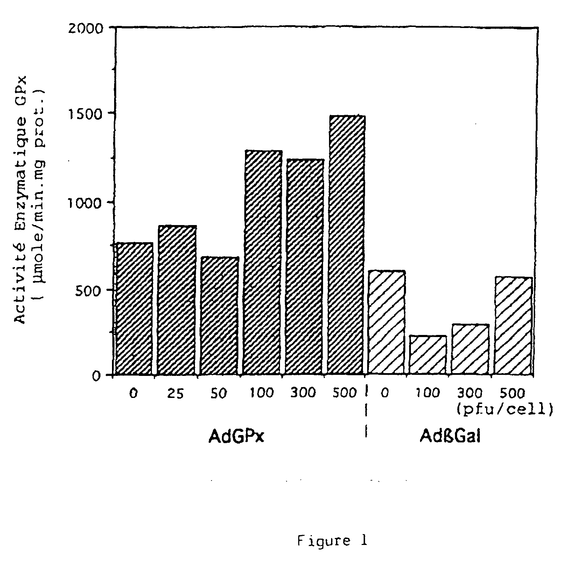

Figure 1: Représentation de l'activité enzymatique de la glutathion peroxydase obtenue sur cellules 293 infectées par 0 à 500 pfu/cellule d'adénovirus recombinant codant pour la GPx ( AdGPx) ou la βgalactosidase ( Adβgal).

### Techniques générales de biologie moléculaire

Les méthodes classiquement utilisées en biologie moléculaire telles que les extractions préparatives d'ADN plasmidique, la centrifugation d'ADN plasmidique en gradient de chlorure de césium, l'électrophorèse sur gels d'agarose ou d'acrylamide, la purification de fragments d'ADN par électroélution, les extraction de protéines au phénol ou au phénol-chloroforme, la précipitation d'ADN en milieu salin par de l'éthanol ou de l'isopropanol, la transformation dans Escherichia coli, etc ... sont bien connues de l'homme de métier et sont abondamment décrites dans la littérature [Maniatis T. et al., "Molecular Cloning, a Laboratory Manual", Cold Spring Harbor Laboratory, Cold Spring Harbor, N.Y., 1982; Ausubel F.M. et al. (eds), "Current Protocols in Molecular Biology", John Wiley & Sons, New York, 1987].

Les plasmides de type pBR322, pUC et les phages de la série M13 sont d'origine commerciale (Bethesda Research Laboratories).

Pour les ligatures, les fragments d'ADN peuvent être séparés selon leur taille par électrophorèse en gels d'agarose ou d'acrylamide, extraits au phénol ou par un mélange phénol/chloroforme, précipités à l'éthanol puis incubés en présence de l'ADN ligase du phage T4 (Biolabs) selon les recommandations du fournisseur.

Le remplissage des extrémités 5' proéminentes peut être effectué par le fragment de Klenow de l'ADN Polymérase I d'E. coli (Biolabs) selon les spécifications du fournisseur. La destruction des extrémités 3' proéminentes est effectuée en présence de l'ADN Polymérase du phage T4 (Biolabs) utilisée selon les recommandations du fabricant. La destruction des extrémités 5' proéminentes est effectuée par un traitement ménagé par la nucléase S1.

La mutagénèse dirigée in vitro par oligodéoxynucléotides synthétiques peut être effectuée selon la méthode développée par Taylor et al. [Nucleic Acids Res. 13 (1985) 8749-8764] en utilisant le kit distribué par Amersham.

L'amplification enzymatique de fragments d'ADN par la technique dite de PCR [Polymérase-catalyzed Chain Reaction, Saiki R.K. et al., Science 230 (1985) 1350-1354; Mullis K.B. et Faloona F.A., Meth. Enzym. 155 (1987) 335-350] peut être effectuée en utilisant un "DNA thermal cycler" (Perkin Elmer Cetus) selon les spécifications du fabricant.

La vérification des séquences nucléotidiques peut être effectuée par la méthode développée par Sanger et al. [Proc. Natl. Acad. Sci. USA, 74 (1977) 5463-5467] en utilisant le kit distribué par Amersham.

### Exemples

### Exemple 1 : Protocole de construction du vecteur pLTRIX-bGPx

Ce vecteur contient la séquence codant pour la GPx bovine sous contrôle du LTR du virus RSV, ainsi que des séquences de l'adénovirus permettant la recombinaison in vivo. L'ADNc utilisé est décrit dans [Mullenbach et al., Oxy-Radicals in Molecular Biology and Pathology, 313-326, (1988)].
L'ADN est inséré dans le site BamHI d'un plasmide Bluescript. Une séquence de polyadénylation a été introduite dans le site XhoI de ce plasmide. Ce dernier est identifié par SK-bGPx-PolyA.
Le vecteur pLTRIX-bGPx est obtenu en introduisant dans le site EcoRV du plasmide pLTRIX un insert obtenu par coupure de SK-bGPx-PolyA

### Exemple 2 : Construction d'adénovirus recombinants contenant une séquence codant pour la glutathion peroxydase bovine.

Le vecteur pLTRIX-bGPx est linéarisé et cotransfecté avec un vecteur adénoviral déficient, dans les cellules helper (lignée 293) apportant en trans les fonctions codées par les régions E1 (E1A et E1B) d'adénovirus.

Plus précisément, l'adénovirus Ad-bGPx a été obtenu par recombinaison homologue in vivo entre l'adénovirus mutant Ad-dl1324 (Thimmappaya et al., Cell 31 (1982) 543) et le vecteur pLTR IX-bGPx, selon le protocole suivant : le plasmide pLTR IX-bGPx et l'adénovirus Ad-dl1324, linéarisé par l'enzyme ClaI, ont été co-transfectés dans la lignée 293 en présence de phosphate de calcium, pour permettre la recombinaison homologue. Les adénovirus recombinants ainsi générés ont été sélectionnés par purification sur plaque. Après isolement, l'ADN de l'adénovirus recombinant a été amplifié dans la lignée cellulaire 293, ce qui conduit à un sumageant de culture contenant l'adénovirus défectif recombinant non purifié ayant un titre d'environ 10¹⁰ pfu/ml.

Les particules virales sont ensuite purifiées par centrifugation sur gradient.

### Exemple 3 : Contrôle de l'expression in vitro de la GPx.

Pour chaque essais, un extrait ( 0,5% triton) est réalisé à partir de 300.000 cellules 293 infectées par 0 à 500pfu/cellule d'adénovirus recombinant codant pour la GPx ou la βgalactosidase. L'activité enzymatique de la glutathion peroxydase est évaluée selon le protocole de Flohé et Günzler ( 1984, Methods in Enzymology, Vol; 105, pp 114-121). Le glutathion oxydé ( GSSG) formé au cours de la réaction GPx est réduit de manière constante par un excès d'activité glutathion réductase pour un niveau constant de glutathion réduit (GSH). L'oxydation simultanée de NADPH est suivie par spectrophotométrie.

La figure 1 rend compte des résultats obtenus.

## Revendications

1. Adénovirus recombinant défectif comprenant au moins une séquence d'ADN codant pour tout ou une partie active d'une glutathion peroxydase.

2. Adénovirus selon la revendication 1 **caractérisé en ce que** la séquence d'ADN est une séquence d'ADNc.

3. Adénovirus selon la revendication 1 **caractérisé en ce que** la séquence d'ADN est une séquence d'ADN génomique.

4. Adénovirus selon la revendication 1, 2 ou 3 **caractérisé en ce que** la séquence d'ADN code pour une glutathion peroxydase bovine.

5. Adénovirus selon la revendication 1, 2 ou 3 **caractérisé en ce que** la séquence d'ADN code pour une glutathion peroxydase humaine.

6. Adénovirus selon la revendication 1 **caractérisé en ce que** la séquence d'ADN est une séquence antisens dont l'expression permet de contrôler l'expression du gène codant pour la glutathion peroxydase.

7. Adénovirus selon la revendication 6 **caractérisé en ce qu'**il s'agit d'un gène codant pour un ARN antisens capable de contrôler la traduction de l'ARNm d'une glutathion peroxydase.

8. Adénovirus selon l'une des revendications 1 à 7 **caractérisé en ce que** la séquence d'ADN est placée sous le contrôle de signaux permettant son expression dans les cellules cibles.

9. Adénovirus selon la revendication 8 **caractérisé en ce que** les signaux d'expression sont choisis parmi les promoteurs viraux, de préférence parmi les promoteurs EIA, MLP, CMV et LTR-RSV.

10. Adénovirus selon la revendication 1 comprenant une séquence d'ADN génomique ou d'ADNc codant pour une glutathion peroxydase bovine sous le contrôle d'un promoteur LTR-RSV.

11. Adénovirus selon la revendication 1 comprenant une séquence d'ADN génomique ou d'ADNc codant pour une glutathion peroxydase humaine sous le contrôle d'un promoteur LTR-RSV.

12. Adénovirus selon l'une des revendications 1 à 11 **caractérisé en ce qu'**il est dépourvu des régions de son génome qui sont nécessaires à sa réplication dans la cellule cible.

13. Adénovirus selon la revendication 12 **caractérisé en ce qu'**il comprend les ITR et une séquence permettant l'encapsidation, et dans lequel le gène E1 et au moins un des gènes E2, E4, L1-L5 sont non fonctionnels.

14. Adénovirus selon la revendication 12 ou 13 **caractérisé en ce qu'**il sagit d'un adénovirus humain de type Ad 2 ou Ad 5 ou canin de type CAV-2.

15. Utilisation d'un adénovirus selon l'une des revendications 1 à 14 pour la préparation d'une composition pharmaceutique destinée au traitement et/ou à la prévention des maladies neurodégénératives.

16. Utilisation d'un adénovirus selon la revendication 15 pour la préparation d'une composition pharmaceutique destinée au traitement et/ou à la prévention de la maladie de Parkinson, de la maladie d'Alzheimer, et de la sclérose latérale amyotrophique (ALS).

17. Composition pharmaceutique comprenant un ou plusieurs adénovirus recombinants défectifs selon l'une des revendications 1 à 15.

18. Composition pharmaceutique selon la revendication 17 **caractérisée en ce qu'**elle est sous forme injectable.

19. Composition pharmaceutique selon l'une des revendications 17 à 18 **caractérisée en ce qu'**elle comprend entre 10⁴ et 10¹⁴ pfu/ml, et de préférence 10⁶ à 10¹⁰ pfu/ml adénovirus recombinants défectifs.

20. Cellule isolée de mammifère infectée par un ou plusieurs adénovirus recombinants défectifs selon l'une des revendications 1 à 14.

21. Cellule selon la revendication 20 **caractérisée en ce qu'**il s'agit d'une cellule humaine.

22. Cellule selon la revendication 21 **caractérisée en ce qu'**il s'agit d'une cellule humaine de type rétinienne, fibroblaste, myoblaste, hépatocyte, cellule endothéliale, cellule Gliales ou kératynocyte.

23. Implant comprenant des cellules infectées selon les revendications 20 à 22 et une matrice extracellulaire.

24. Implant selon la revendication 23 **caractérisé en ce que** la matrice extracellulaire comprend un composé gélifiant choisi de préférence parmi le collagène, la gélatine, les glucosaminoglycans, la fibronectine, l'agarose et les lectines.

25. Implant selon les revendications 23 ou 24 **caractérisé en ce que** la matrice extracellulaire comprend également un support permettant l'ancrage des cellules infectées.

26. Implant selon la revendication 25 **caractérisé en ce que** le support est constitué préférentiellement par des fibres de polytétrafluoroéthylène.

## Patentansprüche

1. Defektes rekombinantes Adenovirus, umfassend wenigstens eine DNA-Sequenz, die für eine ganze oder einen aktiven Teil einer Glutathionperoxidase codiert.

2. Adenovirus gemäß Anspruch 1, **dadurch gekennzeichnet, dass** die DNA-Sequenz eine cDNA-Sequenz ist.

3. Adenovirus gemäß Anspruch 1, **dadurch gekennzeichnet, dass** die DNA-Sequenz eine genomische DNA-Sequenz ist.

4. Adenovirus gemäß Anspruch 1,2 oder 3, **dadurch gekennzeichnet, dass** die DNA-Sequenz für eine bovine Glutathionperoxidase codiert.

5. Adenovirus gemäß Anspruch 1, 2 oder 3, **dadurch gekennzeichnet, dass** die DNA-Sequenz für eine humane Glutathionperoxidase codiert.

6. Adenovirus gemäß Anspruch 1, **dadurch gekennzeichnet, dass** die DNA-Sequenz eine Antisense-Sequenz ist, deren Expression die Kontrolle der Expression des Gens erlaubt, das für die Glutathionperoxidase codiert.

7. Adenovirus gemäß Anspruch 6, **dadurch gekennzeichnet, dass** es sich um ein Gen handelt, das für eine Antisense-RNA codiert, die in der Lage ist, die Translation der mRNA einer Glutathionperoxidase zu kontrollieren.

8. Adenovirus gemäß einem der Ansprüche 1 bis 7, **dadurch gekennzeichnet, dass** die DNA-Sequenz unter der Kontrolle von Signalen steht, die ihre Expression in Zielzellen erlauben.

9. Adenovirus gemäß Anspruch 8, **dadurch gekennzeichnet, dass** die Expressionssignale aus viralen Promotoren, vorzugsweise aus den Promotoren EIA, MLP, CMV und LTR-RSV, ausgewählt sind.

10. Adenovirus gemäß Anspruch 1, umfassend eine genomische DNA- oder cDNA-Sequenz, die für eine bovine Glutathionperoxidase unter der Kontrolle eines LTR-RSV-Promotors codiert.

11. Adenovirus gemäß Anspruch 1, umfassend eine genomische DNA- oder cDNA-Sequenz, die für eine humane Glutathionperoxidase unter der Kontrolle eines LTR-RSV-Promotors codiert.

12. Adenovirus gemäß einem der Ansprüche 1 bis 11, **dadurch gekennzeichnet, dass** es ohne Genombereiche ist, die für seine Replikation in der Zielzelle erforderlich sind.

13. Adenovirus gemäß Anspruch 12, **dadurch gekennzeichnet, dass** es die ITR und eine Sequenz umfasst, die die Verpackung erlauben, und in dem das E1-Gen und wenigstens eines der E2-, E4-, L1-L5-Gene nicht funktionsfähig sind.

14. Adenovirus gemäß Anspruch 12 oder 13, **dadurch gekennzeichnet, dass** es sich um ein humanes Adenovirus vom Typ Ad2 oder Ad5 oder um ein canines Adenovirus vom Typ CAV-2 handelt.

15. Verwendung eines Adenovirus gemäß einem der Ansprüche 1 bis 14 zur Herstellung einer pharmazeutischen Zusammensetzung zur Behandlung und/oder zur Prävention neurodegenerativer Krankheiten.

16. Verwendung eines Adenovirus gemäß Anspruch 15 zur Herstellung einer pharmazeutischen Zusammensetzung zur Behandlung und/oder Prävention der Parkinson Krankheit, der Alzheimer Krankheit und der amyotrophen Lateralsklerose (ALS).

17. Pharmazeutische Zusammensetzung, umfassend ein oder mehrere defekte rekombinante Adenoviren gemäß einem der Ansprüche 1 bis 15.

18. Pharmazeutische Zusammensetzung gemäß Anspruch 17, **dadurch gekennzeichnet, dass** sie in injizierbarer Form ist.

19. Pharmazeutische Zusammensetzung gemäß einem der Ansprüche 17 bis 18, **dadurch gekennzeichnet, dass** sie zwischen 10⁴ und 10¹⁴ PFU/ml und vorzugsweise 10⁶ bis 10¹⁰ PFU/ml defekte rekombinante Adenoviren umfasst.

20. Isolierte Säugerzelle, die mit einem oder mehreren defekten rekombinanten Adenoviren gemäß einem der Ansprüche 1 bis 14 infiziert ist.

21. Zelle gemäß Anspruch 20, **dadurch gekennzeichnet, dass** es sich um eine humane Zelle handelt.

22. Zelle gemäß Anspruch 21, **dadurch gekennzeichnet, dass** es sich um eine humane Zelle wie Retinazelle, Fibroblast, Myoblast, Hepatozyt, Endothelzelle, Gliazelle oder Keratinozyt handelt

23. Implantat, umfassend infizierte Zellen gemäß Ansprüchen 20 bis 22 und eine extrazelluläre Matrix.

24. Implantat gemäß Anspruch 23, **dadurch gekennzeichnet, dass** die extrazelluläre Matrix eine gelbildende Verbindung umfasst, die vorzugsweise aus Kollagen, Gelatine, Glucosaminoglycanen, Fibronektin, Agarose und Lektinen ausgewählt ist.

25. Implantat gemäß Ansprüchen 23 oder 24, **dadurch gekennzeichnet, dass** die extrazelluläre Matrix ebenfalls einen Träger umfasst, der die Verankerung infizierter Zellen erlaubt.

26. Implantat gemäß Anspruch 25, **dadurch gekennzeichnet, dass** der Träger vorzugsweise aus Polytetrafluorethylen-Fasern besteht.

## Claims

1. A defective recombinant adenovirus comprising at least one DNA sequence encoding all or an active part of a glutathione peroxidase.

2. The adenovirus according to claim 1, **characterized in that** the DNA sequence is a cDNA sequence.

3. The adenovirus according to claim 1, **characterized in that** the DNA sequence is a gDNA sequence.

4. The adenovirus according to claim 1, 2 or 3, **characterized in that** the DNA sequence encodes a bovine glutathione peroxidase.

5. The adenovirus according to claim 1, 2 or 3, **characterized in that** the DNA sequence encodes a human glutathione peroxidase.

6. The adenovirus according to claim I, **characterized in that** the DNA sequence is an antisense sequence whose expression makes it possible to control the expression of the gene encoding glutathione peroxidase

7. The adenovirus according to claim 6, **characterized in that** it is a gene encoding an antisense RNA capable of controlling the mRNA translation of a glutathione peroxidase.

8. The adenovirus according to anyone of claims 1 to 7, **characterized in that** the DNA sequence is under the control of signals allowing its expression in target cells.

9. The adenovirus according to claim 8, **characterized in that** the expression signals are selected from the viral promoters, preferably from the E1A, MLP, CMV and RSV-LTR promoters.

10. The adenovirus according to claim 1, comprising a genomic DNA or cDNA sequence encoding a bovine glutathione peroxidase under the control of a RSV LTR promoter.

11. The adenovirus according to claim 1, comprising a genomic DNA or cDNA sequence encoding a human glutathione peroxidase under the control of a RSV LTR promoter.

12. The adenovirus according to anyone of claims 1 to 11, **characterized in that** it is devoid of its genome regions which are necessary for its replication in the target cell.

13. The adenovirus according to claim 12, **characterized in that** it comprises the ITRs and a sequence allowing packaging, and in which the E1 gene and at least one of the E2, E4, L1-L5 genes are non functional.

14. The adenovirus according to claim 12 or 13, **characterized in that** it is an Ad2 or Ad5 type human adenovirus or a CAV-2 type canine adenovirus.

15. Use of the adenovirus according to anyone of claims 1 to 14 for the preparation of a pharmaceutical composition for treating and/or preventing neurodegenerative diseases.

16. Use according to claim 15 for the preparation of a pharmaceutical composition for treating and/or preventing Pakinson's disease, Alzheimer's disease, and amyotrophic lateral sclerosis (ALS).

17. A pharmaceutical composition comprising one or several defective recombinant adenoviruses according to anyone of claims 1 to 15.

18. Pharmaceutical composition according to claim 17, **characterized in that** it is in injectable form.

19. Pharmaceutical composition according to anyone of claims 17 to 18, **characterized in that** it comprises between 10⁴ and 10¹⁴ pfu/ml, preferably between 10⁶ and 10¹⁰ pfu/ml, of defective recombinant adenoviruses.

20. An isolated mammalian cell infected by one or several defective recombinant adenoviruses according to anyone of claims 1 to 14.

21. Cell according to claim 20, **characterized in that** the cell is a human cell.

22. Cell according to claim 21, **characterized in that** the cell is a human cell of the retinal, fibroblast, myoblast, hepatocyte, endothelial cell, glial cell or keratinocyte type.

23. An implant comprising infected cells according to claims 20 to 22 and an extracellular matrix.

24. Implant according to claim 23, **characterized in that** the extracellular matrix comprises a gelling compound chosen preferably from collagen, gelatine, glucosaminoglycans, fibronectin, agarose and lectins.

25. Implant according to claims 23 and 24, **characterized in that** the extracellular matrix also comprises a support allowing anchorage of the infected cells.

26. Implant according to claim 25, **characterized in that** the support consists preferably of polytetrafluoroethylene fibres.
